# EUROPEAN PATENT APPLICATION

(11) **EP 4 086 230 A1**
(43) Date of publication of application: **09.11.2022**
(21) Application number: 21192974.0
(22) Date of filing: 25.08.2021
(51) Int. Cl.: C02F 1/467, A61L 2/00

(54) **NON-DIAPHRAGM NEUTRAL STERILIZING WATER GENERATING APPARATUS**

(30) Priority: 07.05.2021 KR 20210059122
(71) Applicant: Ecobm Co., Ltd., Jeollanam-do 58034 (KR)
(72) Inventor: KIM, Young Sun, 57953 Suncheon-si, Jeollanam-do (KR)
(74) Representative: BCKIP Part mbB

(57) **Abstract**

Provided is a non-diaphragm neutral sterilizing water generating apparatus including: a washing water tank in which a predetermined inner space is formed to accommodate salt water or washing water and into which an object to be washed is input; an electrolyzed water tank having a structure into which the salt water or washing water for washing the object to be washed is introduced and through which electrolyzed water containing a hypochlorous acid (HOC1) solution generated by electrolyzing the salt water or washing water is discharged; and at least one unit cell accommodated in the electrolyzed water tank and including a positive electrode unit and a negative electrode unit, wherein the unit cell has a structure in which at least two electrolytic cells each having protruding positive and negative electrodes are stacked on each other at a predetermined interval.

## Description

### [Technical Field]

The present disclosure relates to a non-diaphragm neutral sterilizing water generating apparatus, and more particularly, to a non-diaphragm neutral sterilizing water generating apparatus capable of generating high-concentration electrolyzed water with higher efficiency.

### [Background Art]

In general, sodium hypochlorite may be used as a sterilizer killing spoilage bacteria or pathogens in food, used for sterilizing and washing fruits, vegetables, tableware and the like, and used widely in various workplaces such as a water purification plant, a sewage treatment plant, a general chemical plant facility, a swimming pool, etc.

Sterilizing water containing the sodium hypochlorite may be generated by electrolyzing water containing a salt solution. When water with a constant salt (NaCl) concentration passes through between negative and positive electrode plates, chlorine ions (Cl⁻) may be oxidized to become chlorine (Cl₂) at the positive electrode, and sodium ions (Na⁺) may be reduced at the negative electrode to become sodium (Na). The reduced sodium (Na) may react with water (H₂O) to generate sodium hydroxide (NaOH) and hydrogen (H₂), and chlorine (Cl₂) generated at the positive electrode and sodium hydroxide (NaOH) generated at the negative electrode react with each other to generate sodium hypochlorite (NaOCl).

However, in recent years, there is an increasing demand for near-neutral sterilizing water which is relatively harmless to a human body as sterilizing electrolyzed water.

### [Related Art Document]

(Patent Document 1) Korean Patent Publication No. 10-1904840
(Patent Document 2) Korean Patent Publication No. 10-1738124

### [Disclosure]

### [Technical Problem]

An object of the present disclosure is to provide a non-diaphragm neutral sterilizing water generating apparatus capable of generating electrolyzed water containing a high-concentration hypochlorous acid (HOCl) solution, i.e. hypochlorous acid water, which is relatively harmless to a human body, by electrolyzing water containing a salt solution using a unit cell capable of high-capacity output by including a plurality of electrolytic cells.

In addition, an object of the present disclosure is to provide a non-diaphragm neutral sterilizing water generating apparatus having a flow path in which salt water introduced into an electrolyzed water tank storing the unit cell may be efficiently electrolyzed.

In addition, an object of the present disclosure is to provide a non-diaphragm neutral sterilizing water generating apparatus which may be easily installed and moved by allowing the unit cells in a stacked form to be independently stored in a housing, i.e. inner space of the apparatus.

In addition, an object of the present disclosure is to provide a non-diaphragm neutral sterilizing water generating apparatus capable of generating near-neutral electrolyzed water (hypochlorous acid water) which is relatively harmless to the human body by containing the high-concentration hypochlorous acid (HOCl) solution and excluding hydrogen chloride (HCl).

### [Technical Solution]

In one aspect, the present disclosure provides a non-diaphragm neutral sterilizing water generating apparatus including:
a washing water tank in which a predetermined inner space is formed to accommodate salt water or washing water and into which an object to be washed is input;
an electrolyzed water tank having a structure into which the salt water or washing water for washing the object to be washed is introduced and through which electrolyzed water containing a hypochlorous acid (HOCl) solution generated by electrolyzing the salt water or washing water is discharged; and
at least one unit cell accommodated in the electrolyzed water tank and including a positive electrode unit and a negative electrode unit, wherein
the unit cell has a structure in which
at least two electrolytic cells each having protruding positive and negative electrodes are stacked on each other at a predetermined interval.

In one aspect, the positive electrode unit and negative electrode unit of the unit cell each may protrude in one direction of the electrolyzed water tank.

The positive electrode and negative electrode of the electrolytic cell may be electrically connected to the positive electrode unit and negative electrode unit of the unit cell, respectively.

In one aspect, the electrolyzed water tank may include:
an inlet into which the salt water or washing water is introduced; and
an outlet through which the electrolyzed water containing a hypochlorous acid (HOCl) solution generated by electrolyzing the salt water or washing water is discharged.

In one aspect, the inlet may be formed above the outlet.

In one aspect, the at least one inlet may be formed on a portion opposite to each of two sides of the electrolyzed water tank.

In one aspect, the outlet may have a filter device.

In one aspect, the electrolyzed water containing the hypochlorous acid (HOCl) solution filtered from the filter device may be introduced into the washing water tank or re-introduced into the electrolyzed water tank.

In one aspect, the high-concentration hypochlorous acid (HOCl) solution may be generated by adding an electrolyte of sodium chloride (NaCl), potassium chloride (KCl), sodium hydroxide (NaOH) or potassium hydroxide (KOH) to the electrolyzed water tank.

In one aspect, the electrolyzed water tank may have a bus bar to which the positive electrode and negative electrode of the electrolytic cell are respectively connected, the bus bar being electrically connected to each of the positive electrode unit and negative electrode unit of the unit cell.

In one aspect, the electrolytic cells of the unit cell may be respectively connected to and removed from the bus bar independently from each other.

In one aspect, the positive electrode and negative electrode of the electrolytic cell may be vertically bent, respectively, and the bus bar may have a detachable groove for the bent portion to be detached therefrom.

In one aspect, the electrolytic cell may be inserted into and removed from the bus bar in a sliding manner in one direction.

In one aspect, a vertically-extending fixing plate may be interposed between the electrolytic cells.

In one aspect, the fixing plate may have a plurality of flow holes formed at a predetermined interval to allow the electrolyzed water containing the hypochlorous acid (HOCl) solution to flow therethrough.

In one aspect, the fixing plate may have a filter.

In one aspect, the electrolyzed water tank may have a structure in which a pair of upper and lower cases are coupled to each other.

In one aspect, a sealing member sealing the upper and lower cases to each other may be formed between bonding surfaces of the cases.

In one aspect, the hypochlorous acid (HOCl) solution generated by the electrolysis performed by the unit cell may have a concentration of 300 to 1500 ppm.

### [Advantageous Effect]

The present disclosure provides the non-diaphragm neutral sterilizing water generating apparatus capable of generating the electrolyzed water containing a high-concentration hypochlorous acid (HOCl) solution, i.e. hypochlorous acid water, by electrolyzing the water containing a salt solution using the unit cell capable of high-capacity output by including the plurality of electrolytic cells.

The present disclosure provides the non-diaphragm neutral sterilizing water generating apparatus having the flow path in which the salt water or washing water introduced into the electrolyzed water tank storing the unit cell may be efficiently electrolyzed.

The present disclosure provides the non-diaphragm neutral sterilizing water generating apparatus which may be easily installed and moved by allowing the unit cells in a stacked form to be independently stored in the housing, i.e. inner space of the apparatus.

The present disclosure provides the non-diaphragm neutral sterilizing water generating apparatus capable of generating the near-neutral electrolyzed water (hypochlorous acid water) which is relatively harmless to the human body by containing the high-concentration hypochlorous acid (HOCl) solution and excluding the hydrogen chloride (HCl).

### [Description of Drawings]

FIG. 1 is a perspective view showing an electrolyzed water tank of a non-diaphragm neutral sterilizing water generating apparatus according to an embodiment of the present disclosure.
FIGS. 2A and B are views each showing the electrolyzed water tank of the non-diaphragm neutral sterilizing water generating apparatus according to an embodiment of the present disclosure.
FIG. 3 is a schematic configuration diagram of the non-diaphragm neutral sterilizing water generating apparatus according to an embodiment of the present disclosure.
FIGS. 4 to 6 are views each showing an electrolytic cell of the non-diaphragm neutral sterilizing water generating apparatus according to an embodiment of the present disclosure.

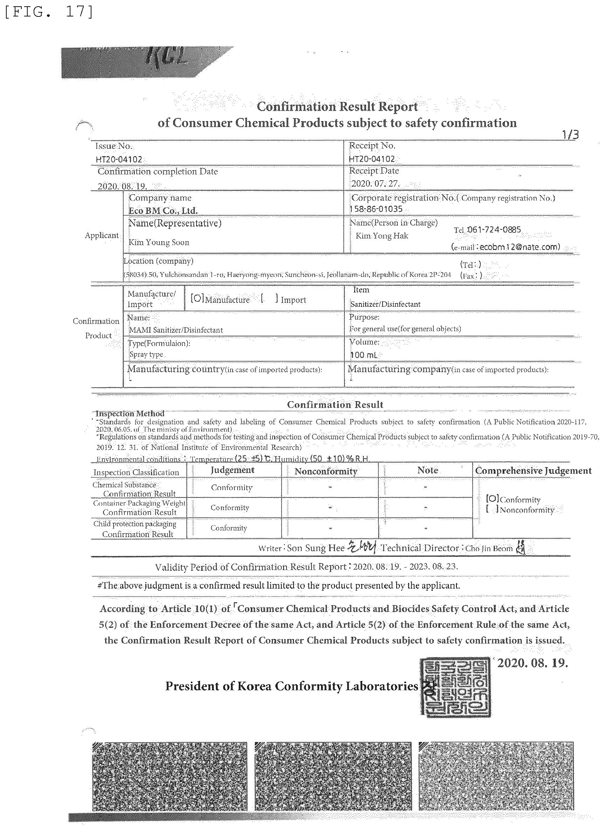

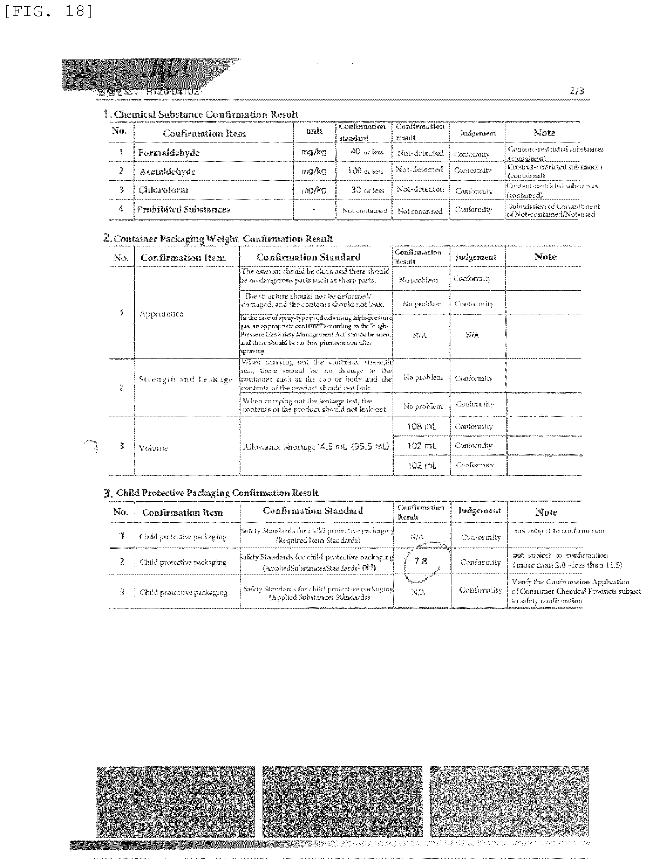

FIGS. 7 to 18 show the respective test and inspection reports for electrolyzed water of the non-diaphragm neutral sterilizing water generating apparatus according to an embodiment of the present disclosure.

### [Best Mode]

Hereinafter, an embodiment of the present disclosure is described in detail with reference to the accompanying drawings. It is to be noted that in giving reference numerals to components of the accompanying drawings, the same components are denoted by the same reference numerals even though they are illustrated in different drawings. Further, in describing an embodiment of the present disclosure, well-known constructions or functions are not described in detail in a case in which it is decided that they may unnecessarily obscure the understanding of an embodiment of the present disclosure.

Terms 'first' , 'second' , A, B, (a), (b) and the like, are used in describing components of an embodiment of the present disclosure. These terms are used only in order to distinguish any components from other components, and features, sequences or the like of the corresponding components are not limited by these terms. In addition, unless defined otherwise, all the terms used in the present specification, including technical and scientific terms, have the same meanings as meanings that are generally understood by those skilled in the art to which the present disclosure pertains. It should be interpreted that terms defined by a generally used dictionary have the same meanings as the meanings in the context of the related art, and these terms should not be ideally or excessively formally interpreted unless the context clearly dictates otherwise.

As described above, the present disclosure may provide neutral sterilizing water that may not only be relatively harmless to a human body compared to conventional sterilizing water, but may also generate a smaller amount of sodium hydroxide (NaOH) than conventional strongly alkaline electrolyzed water prepared by adding additional salt, by excluding hydrogen chloride (HCl) and only using water and salt water to generate the neutral sterilizing water.

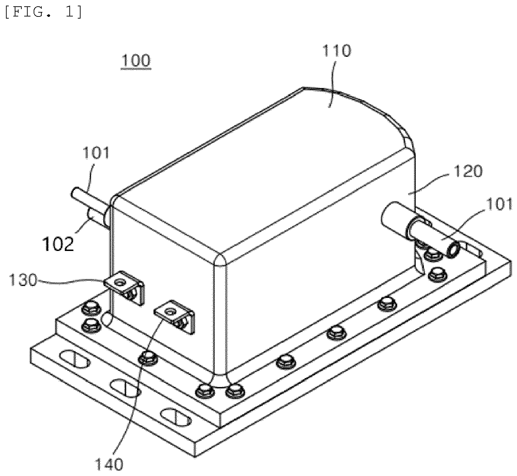

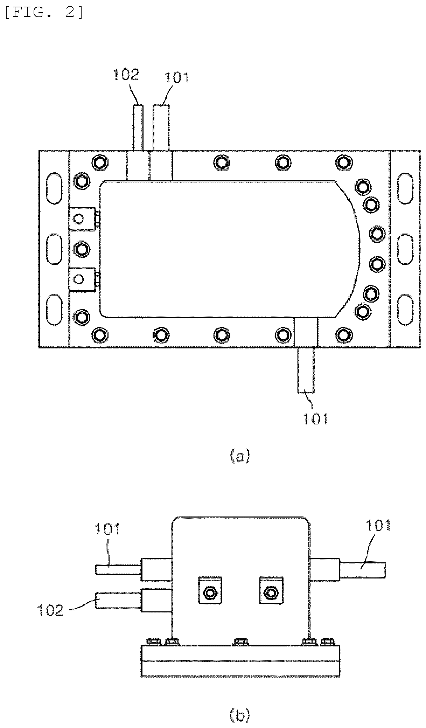

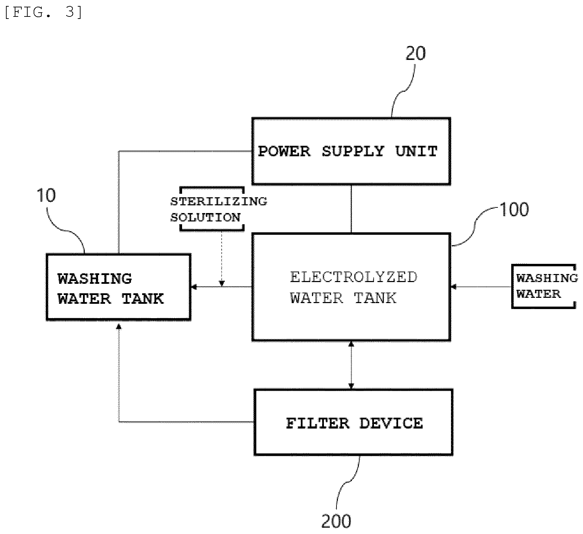

FIG. 1 is a perspective view showing an electrolyzed water tank of a non-diaphragm neutral sterilizing water generating apparatus according to an embodiment of the present disclosure; FIGS. 2A and B are views each showing the electrolyzed water tank of the non-diaphragm neutral sterilizing water generating apparatus according to an embodiment of the present disclosure; and FIG. 3 is a schematic configuration diagram of the non-diaphragm neutral sterilizing water generating apparatus according to an embodiment of the present disclosure.

Referring to the drawings, according to an embodiment of the present disclosure, the non-diaphragm neutral sterilizing water generating apparatus capable of generating electrolyzed water containing a hypochlorous acid (HOCl) solution using an electrolytic method may include: an electrolyzed water tank 100, a washing water tank 10, a filter device 200, a power supply unit 20 and a control unit (not shown).

The washing water tank 10 may allow washing water to be introduced into its inner chamber through an inlet (not shown) and sprayed through a plurality of spray nozzles (not shown) installed therein to effectively wash an object to be washed in the inner chamber.

The object to be washed may be input into the washing water tank 10 using a separate tray (not shown) or a net, and the washing water introduced through the inlet may allow a unit cell 300 of the electrolyzed water tank 100 described below to be fully submerged therein. Here, it is preferable to determine an input amount of the object to be washed in consideration of a volume of the object to be washed, a volume of the washing water tank 10 and a salt elution amount. In addition, the object to be washed may be washed by an internal circulation of sterilizing water through the high-pressure spray nozzles installed at the bottom or side of the electrolyzed water tank 10.

An outlet may be installed at the bottom of the washing water tank 10, and the washed object to be washed and the electrolyzed water containing a hypochlorous acid (HOCl) solution may be discharged through the outlet. The washing water tank 10 may have an open top portion for washing of the object to be washed.

Meanwhile, the electrolyzed water tank 100 according to the present disclosure may have a space accommodating the unit cell 300, the space having a structure in which a pair of upper and lower cases 110 and 120 are coupled to each other. Alternatively, in some cases, the electrolyzed water tank 100 may be configured as an independent space partitioned from the washing water tank 10 by a separate diaphragm (not shown). As shown in FIG. 1, the lower case 120 of the electrolyzed water tank 100 may have a plurality of bolts screwed and released, and may thus be installed or removed by moving its installation location if necessary.

The electrolyzed water tank 100 may have a pair of inlets 101 into which the salt water or washing water is introduced and a pair of outlets 102 through which the hypochlorous acid (HOCl) solution generated by electrolyzing the salt water or washing water is discharged. It may be preferable that the pair of inlets 101 are formed at both upper ends of the electrolyzed water tank 100 for the washing water to be quickly introduced into the electrolyzed water tank 100.

Here, it may be preferable that the pair of outlets 102 are respectively formed below the pair of inlets 101, as shown in FIG. 2B. That is, the hypochlorous acid (HOCl) solution generated by eluting the salt water or washing water introduced into the inlet 101 may be discharged more easily by hydraulic pressure.

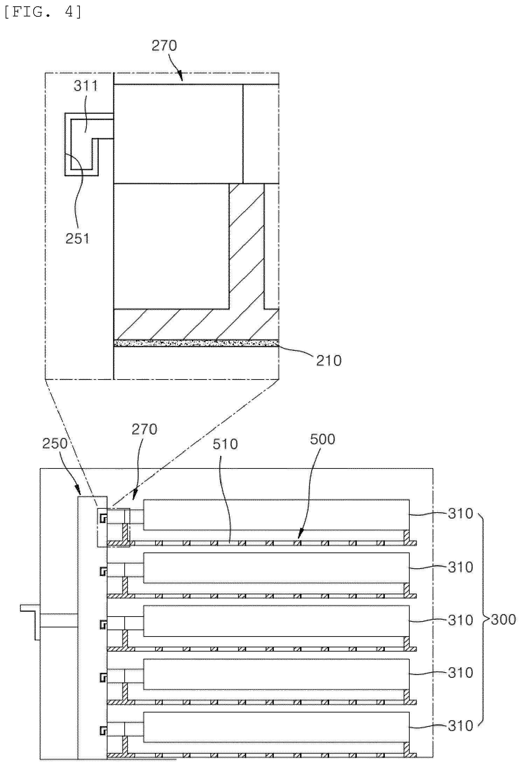

In order to generate the hypochlorous acid (HOCl) water by electrolyzing the salt water or washing water, the unit cell 300 may be disposed in the inner space of the electrolyzed water tank 100, the unit cell 300 having a structure in which a plurality of electrolytic cells (310 in FIG. 4) are stacked on each other. The description below describes this structure of the unit cell 300 in more detail.

The filter device 200 may be disposed on one side of the electrolyzed water tank 100. The electrolyzed water of the electrolyzed water tank 100, containing a hypochlorous acid (HOCl) solution may be directly introduced into the washing water tank 10 or re-introduced into the electrolyzed water tank 100, through the filter device 200. Here, the high-concentration hypochlorous acid (HOCl) solution may be generated by adding an electrolyte of sodium chloride (NaCl), potassium chloride (KCl), sodium hydroxide (NaOH) or potassium hydroxide (KOH) to the electrolyzed water tank 100.

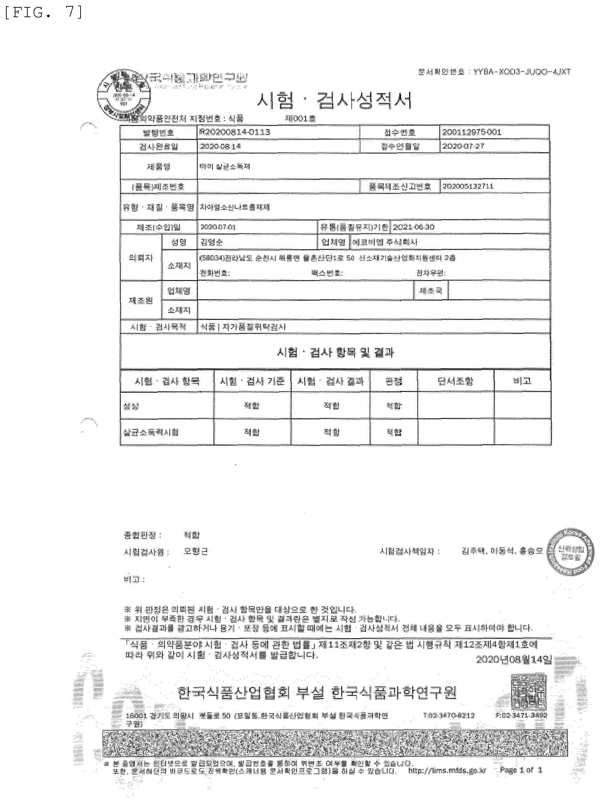

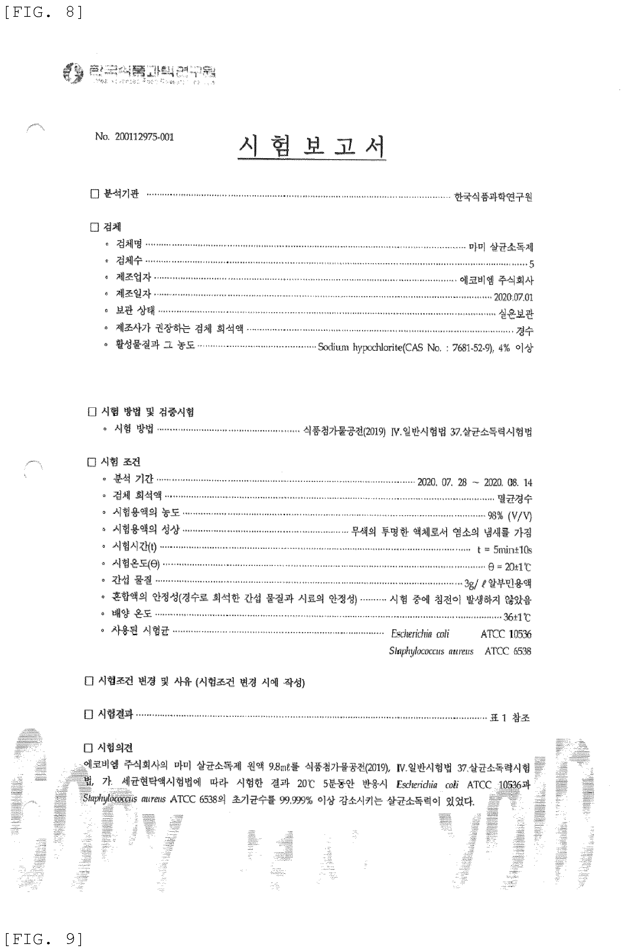

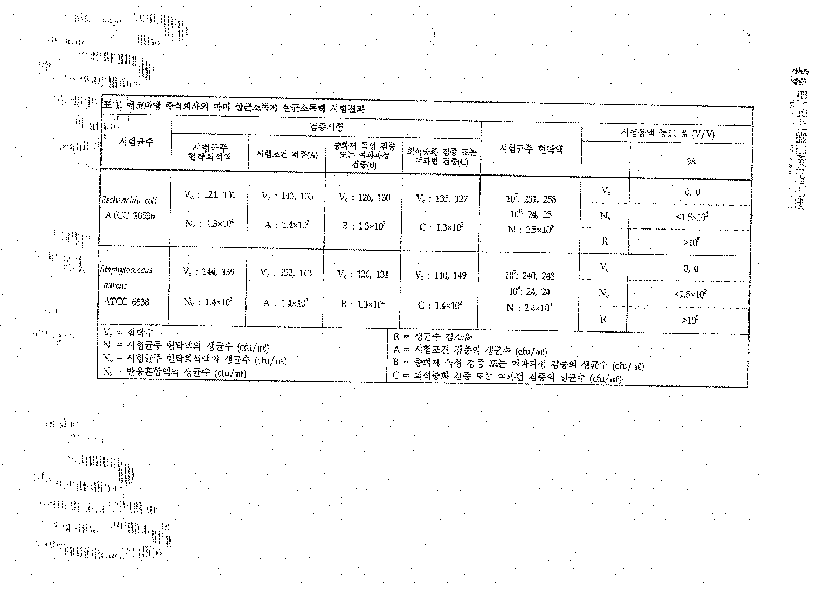

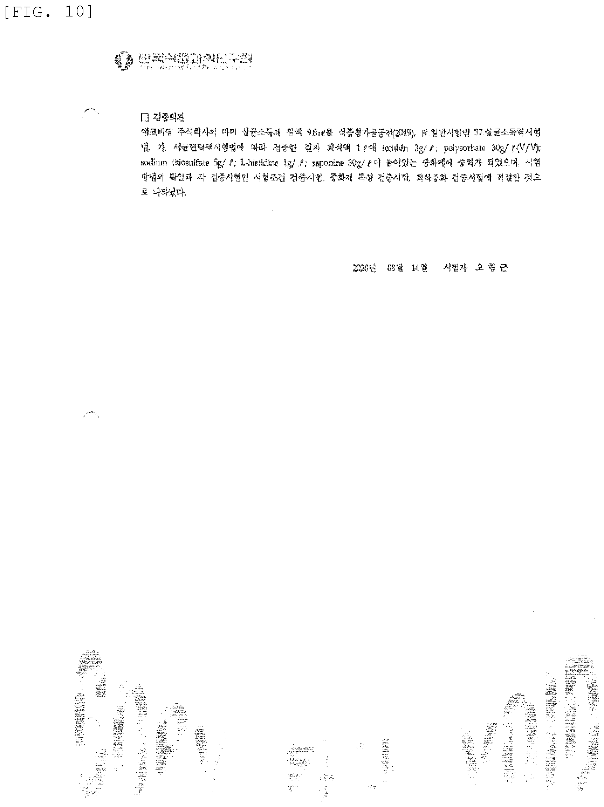

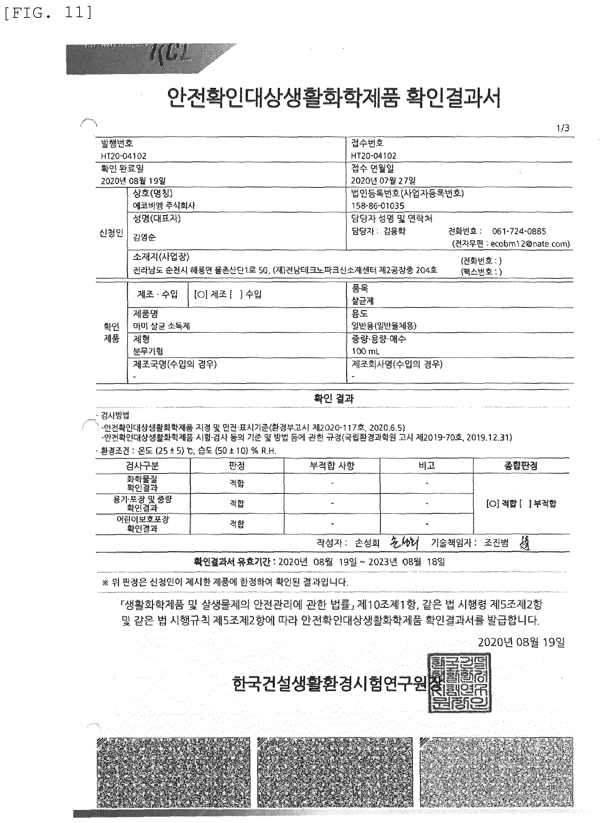

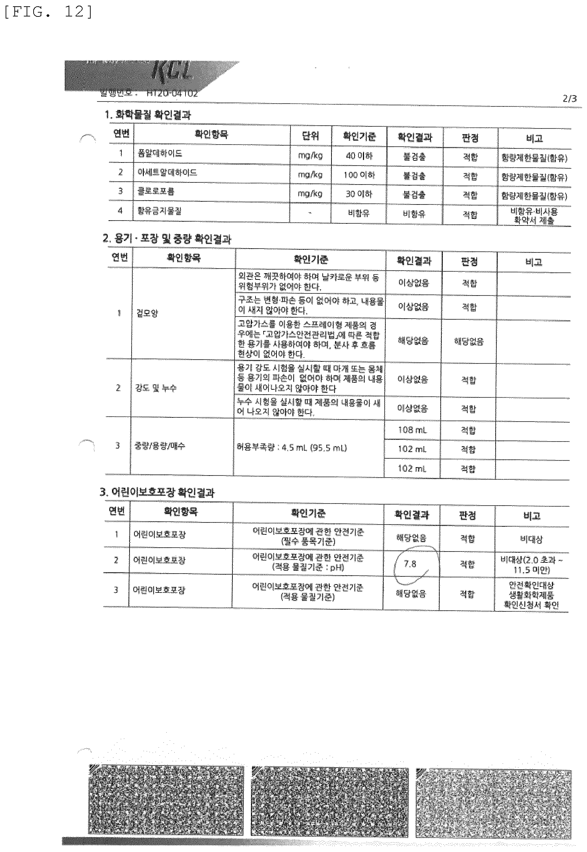

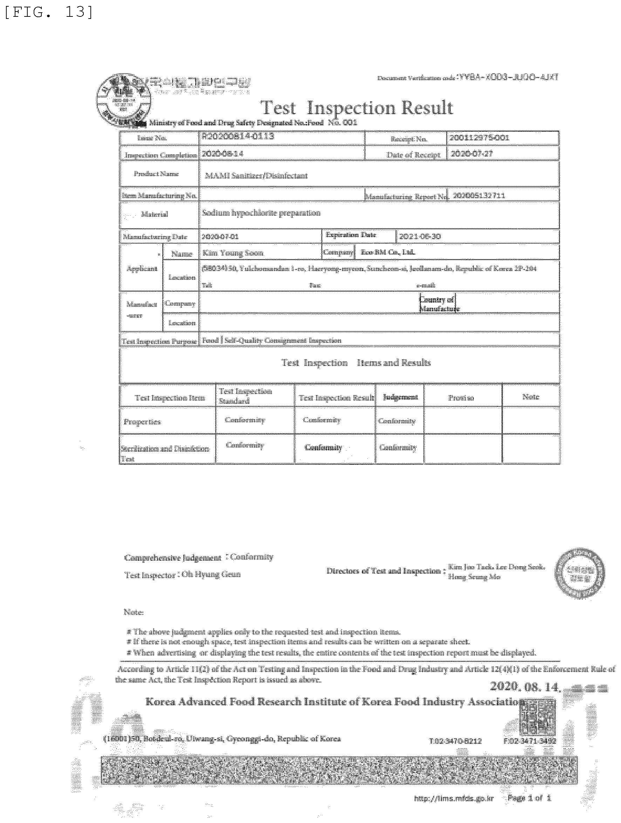

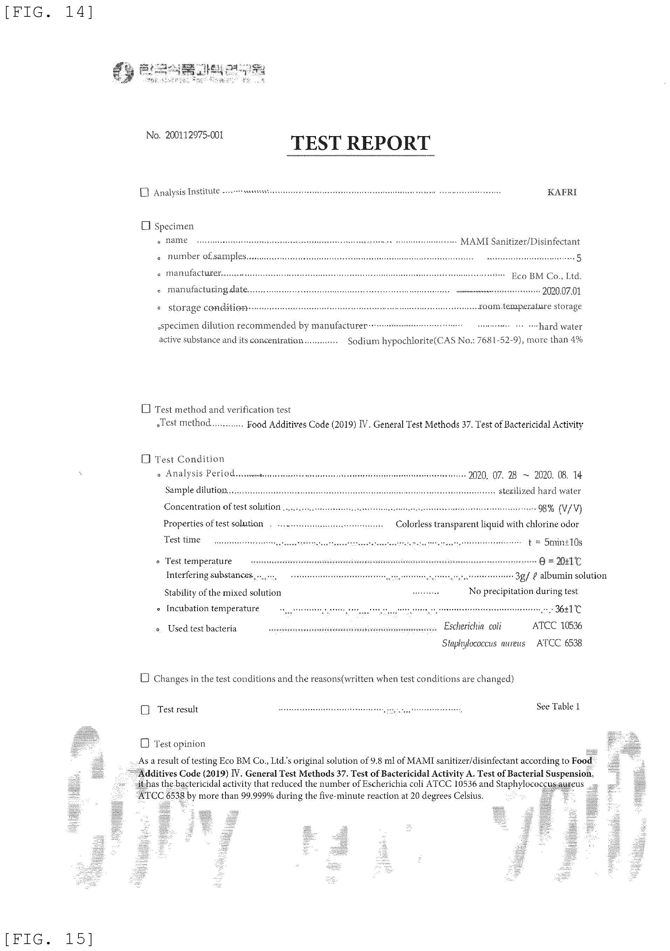

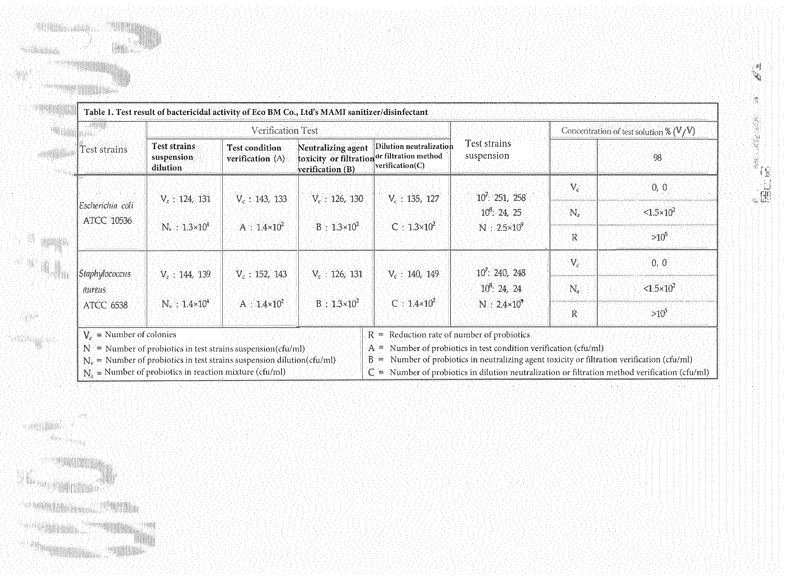

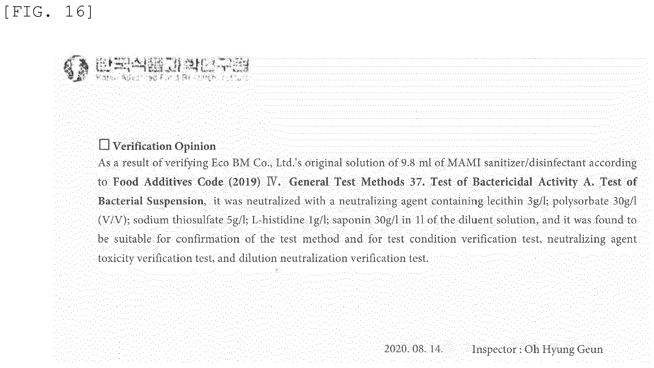

In some cases, a sensor (not shown) measuring a potential of hydrogen (pH) may be installed in the electrolyzed water tank 100, and it is thus possible to measure a concentration of the generated high-concentration hypochlorous acid (HOCl) solution. In one example, the electrolyzed water introduced into the washing water tank 10 may be automatically or manually adjusted by allowing its pH change occurring in the electrolyzed water tank 100 to be detected in real time to suppress an excessive rise in its pH during an electrolysis process. According to the present disclosure, it may be preferable that the pH of the electrolyzed water in the electrolyzed water tank 100 is maintained at a slightly acidic or near-neutral state in a range of approximately 6 to 8. As shown in FIGS. 7 to 16, it may be confirmed that the electrolyzed water generated by a non-diaphragm neutral sterilizing water generating apparatus 100 according to the present disclosure shows a near-neutral pH of approximately 7.8.

That is, as shown in test results of FIGS. 7 to 16, it is possible to confirm a verification opinion that the electrolyzed water produced by the non-diaphragm neutral sterilizing water generating apparatus 100 according to the present disclosure is suitable for sterilization and disinfection. The reason is that a test strain is confirmed to be effectively neutralized also in a result of the sterilization and disinfection test for the test strain after performing the sterilization by the electrolyzed water.

In addition, a safety test of the electrolyzed water not only confirms that none of prohibited substances including formaldehyde, acetaldehyde, chloroform and the like, is detected from the electrolyzed water, but confirms that the package, container and the like of the electrolyzed water are also safe.

Therefore, the electrolyzed water may provide the sterilizing water harmless to the human body by maintaining its near-neutral component.

In one example, the non-diaphragm neutral sterilizing water generating apparatus 100 may generate specific-concentration electrolyzed water using a concentration (ppm) of the hypochlorous acid (HOCl) solution in the electrolyzed water.
That is, when a desired concentration (ppm) is input as a target concentration of the electrolyzed water to be generated, and a target production volume (liter) to be produced is set and input, the non-diaphragm neutral sterilizing water generating apparatus 100 according to the present disclosure may automatically generate the electrolyzed water having the desired target concentration based on the set value.

In addition, a detection sensor (not shown) may be installed in the filter device 200. In this case, after the concentration of the electrolyzed water containing a hypochlorous acid (HOCl) solution is measured, the electrolyzed water may be introduced into either the washing water tank 10 or the electrolyzed water tank 100 based on the concentration. That is, based on a predetermined reference value of the concentration of the hypochlorous acid (HOCl) solution, the electrolyzed water may be directly introduced into the washing water tank 10 when the concentration of the hypochlorous acid (HOCl) solution has a value more than or equal to the reference value, and may be re-introduced into the electrolyzed water tank 100 when the concentration of the hypochlorous acid (HOCl) solution has a value less than the reference value.

In addition, the direct current (DC) power supply unit 20 may be installed on one side of the electrolyzed water tank 100. Here, an electrolysis reaction may occur by the power supply
unit 20 under constant current or constant voltage conditions. According to the present disclosure, in one example, the hypochlorous acid (HOCl) solution may be generated by applying current density within 1 to 15 ampere per square decimeter (ASD), preferably within 3 to 10 ASD. In addition, the hypochlorous acid (HOCl) solution may be generated by the power supply unit 20 when power of about 80 V is applied thereto and operated for 30 to 60 minutes. Electrolysis operation time may be within 10 to 60 minutes, preferably 20 to 40 minutes in consideration of efficiency and economy.

Therefore, the electrolytic cell 310 may be operated by the power supply unit 20 for 30 to 60 minutes as described above, thereby allowing the electrolyzed water containing a hypochlorous acid (HOCl) solution to be continuously sprayed to the object to be washed at high pressure. In this manner, the object to be washed may be rubbed and washed.

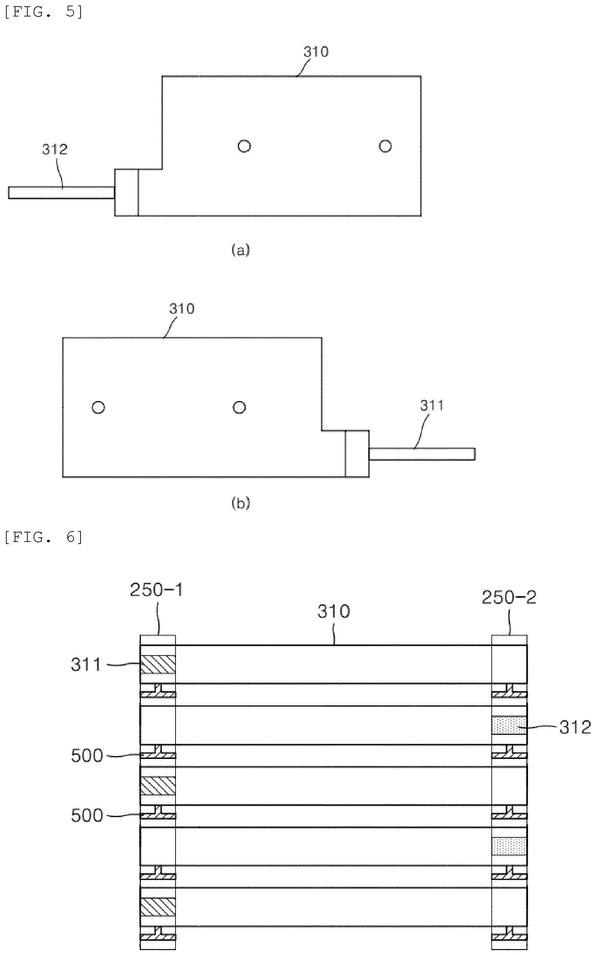

FIGS. 4, 5A and 5B are schematic views each showing the unit cell including the electrolytic cell of the non-diaphragm neutral sterilizing water generating apparatus according to an embodiment of the present disclosure.

Referring to the drawings, the above-described electrolyzed water tank 100 of the non-diaphragm neutral sterilizing water generating apparatus 100 according to the present disclosure may store the unit cell 300 formed by vertically stacking the plurality of electrolytic cells 310 on each other. Here, the electrolytic cell 310 may include a positive electrode 312 and a negative electrode 311, may be installed in the electrolyzed water tank 100, and may electrolyze the salt water or washing water accommodated in the electrolyzed water tank 100 to generate the electrolyzed water containing a hypochlorous acid (HOCl) solution.

This unit cell 300 may have a structure in which the plurality of electrolytic cells 310 are stacked on each other in the vertical direction, and positive electrodes 312 and negative electrodes 311 of the plurality of electrolytic cells 310 each extend in one direction, i.e. direction toward a bus bar 250, while being connected to a terminal unit 270, and are electrically connected to each other. The bus bar 250 may include a first bus bar 250-1 and a second bus bar 250-2 to which the positive electrode 312 and negative electrode 311 of the electrolytic cell 310 are respectively connected. The first bus bar 250-1 and the second bus bar 250-2 may each be connected to the negative electrode unit 140 and positive electrode unit 130, each extending to the outside of the electrolyzed water tank 100. Therefore, the high-concentration hypochlorous acid (HOCl) solution may be generated by the high-capacity unit cell 300 having the structure in which the plurality of electrolytic cells 310 are stacked on each other.

Here, the electrolytic cell 310 may have a non-diaphragm structure and thus be detachably installed in the electrolyzed water tank 100 in an open type. Oxygen and hydrogen gas generated from the electrode units may still be smoothly discharged, and reaction heat may be discharged by their rapid circulations to the electrolyzed water tank 100. In a specific example, the positive electrode 312 and the negative electrode 311, which are electrode units of the electrolytic cell 310, may be formed in a plate shape or a mesh shape, and may have an inter-electrode distance of 1 to 10 mm or 3 to 6 mm.

In addition, the positive electrode 312 and negative electrode 311 of the electrolytic cell 310 may be detachably coupled to a detachable groove 251 formed in each bus bar 250 in a sliding manner. Therefore, each corresponding electrolytic cell 310 may be replaced and repaired independently from each other. That is, when the electrode in use is required to be washed due to a scale, only the corresponding electrolytic cell 310 may be easily removed and washed.

This coupling method may be changed based on an electrolytic sterilization apparatus standard and an electrolytic cell standard.

The positive electrode 312 of the electrolytic cell 310 may use a substrate made of titanium, and may be manufactured by coating titanium, tin, tantalum, niobium, zirconium or the like as an additive to a platinum (Pt)-based compound, e.g., platinum, iridium, palladium, rhodium or ruthenium. In addition, a special coating may be performed on the electrode to prevent a deteriorated lifespan of the electrode occurring due to an acid washing performed to remove the scale thereon, and the composition of a metal catalyst active material may be designed for electrolysis efficiency to be excellent even when the electrolyte has a low chlorine concentration.

The negative electrode 311 of the electrolytic cell 310 may use a substrate made of titanium, hastelloy or nickel, or may be an electrode plated with Pt series on the substrate or an electrode plated with nickel on carbon steel.

In addition, a fixing plate 500 supporting a lower portion of the electrolytic cell 310 may be interposed between the plurality of electrolytic cells 310 of the unit cell 300. The fixing plate 500 may have a flow hole 510 therein to allow the washing water and the hypochlorous acid (HOCl) solution to flow smoothly. That is, the fixing plate 500 according to the present disclosure may be formed across both end portions of the plurality of electrolytic cells 310 in the longitudinal direction and have the flow hole 510 to ensure contact time of the electrolytic cell 310 with the washing water and hypochlorous acid (HOCl) solution without disturbing the flow of the washing water and hypochlorous acid (HOCl) solution, and to generate the electrolyzed water with high efficiency. Accordingly, the fixing plate 500 may further have a filter 210 to filter out a foreign material from the washing water.

The present disclosure is described in more detail through the following Inventive Examples, and the scope of the present disclosure is not limited thereto.

### [Inventive Example]

### [Inventive Examples A to D]

A foreign material is simply removed from a fruit and vegetable, and the fruit and vegetable is input into the washing water tank 10. About 100L of salt water is input into the electrolyzed water tank 100 for the unit cell 300 in the electrolyzed water tank 100 to be sufficiently submerged. Here, based on an installation environment, five (5) or more of the electrolytic cells 310 included in the unit cell 300 are stacked on one another to have high-capacity output, and the fixing plate 500 having the flow hole 510 is interposed between the electrolytic cells 310.

After the water is input, the fruit and vegetable is washed for 10 minutes at a flow rate of 200 L/min using a circulation pump (not shown). The hypochlorous acid (HOCl) solution having a concentration of 600 ppm or more is generated by applying 5V under a constant voltage condition at room temperature, and performing the electrolytic reaction for 30 minutes. First, water and the sodium chloride (NaCl) solution are mixed with each other to generate a mixed solution in which the hypochlorous acid (HOCl) solution and a small amount of sodium hypochlorite (NaOCl) are mixed with each other according to the hydrogen group (H⁺) of the water and the chlorine group (Cl⁻) of sodium chloride (NaCl), thereby generating the electrolyzed water having near-neutral pH of approximately 7.8.

Here, the generated hypochlorous acid (HOCl) solution is sprayed on the fruit and vegetable at high pressure in real time to remove organic material and the like therefrom, and the concentration of hypochlorous acid (HOCl) solution is measured based on the electrolysis reaction time.

### [Comparative Example]

Under the same conditions as in the Inventive Examples, the salt water is electrolyzed using the fixing plate 500 having no flow hole, and the concentration of hypochlorous acid (HOCl) solution is measured.

**[Table 1]**

| Sample | | Object to be washed (kg) | Salt water dosage (L) | Hypochlorous acid (HOCl) water (ppm) | | |
|---|---|---|---|---|---|---|
| | | | | 10 min. | 20 min. | 30 min. |
| Inventive Example | A | 20 | 100 | 170 | 350 | 650 |
| | B | 20 | 100 | 170 | 340 | 610 |
| | C | 20 | 100 | 180 | 580 | 780 |
| | D | 25 | 100 | 190 | 610 | 770 |
| Comparative Example | | 25 | 100 | 170 | 460 | 510 |

Referring to [Table 1] above, a salt concentration of the water is measured based on the input amount of the fruit and vegetable, and the amount of salt (g) is calculated based on 100L of the water input. As a result of the electrolysis, it is confirmed that the concentration of hypochlorous acid (HOCl) solution increases as time passes.

In addition, as in Inventive Examples A to D, when the fruit and vegetable of 20 kg or 25 kg is input into the washing water tank 10, and the electrolytic reaction is performed for 30 minutes, the hypochlorous acid (HOCl) solution having a concentration of 600 ppm or more is generated.

As shown in the Inventive Examples in Table 1 above, it is confirmed that the higher-concentration hypochlorous acid (HOCl) solution is generated when using the high-capacity unit cell according to the present disclosure, which includes five electrolytic cells stacked on one another. In particular, Inventive Example C shows that the high-concentration hypochlorous acid (HOCl) solution of about 30% or more is generated in 30 minutes, compared to the Comparative Example.

As described above, the present disclosure is described with reference to the specific embodiments. However, the present disclosure is not limited thereto, and may be variously modified and altered by those skilled in the art to which the present disclosure pertains without departing from the spirit and scope of the present disclosure claimed in the claims.

## Claims

1. A non-diaphragm neutral sterilizing water generating apparatus comprising:
a washing water tank in which a predetermined inner space is formed to accommodate salt water or washing water and into which an object to be washed is input;
an electrolyzed water tank having a structure into which the salt water or washing water for washing the object to be washed is introduced and through which electrolyzed water containing a hypochlorous acid (HOCl) solution generated by electrolyzing the salt water or washing water is discharged; and
at least one unit cell accommodated in the electrolyzed water tank and including a positive electrode unit and a negative electrode unit,
wherein the unit cell has a structure in which at least two electrolytic cells each having protruding positive and negative electrodes are stacked on each other at a predetermined interval.

2. The non-diaphragm neutral sterilizing water generating apparatus of claim 1, wherein the positive electrode unit and negative electrode unit of the unit cell each protrude in one direction of the electrolyzed water tank.

3. The non-diaphragm neutral sterilizing water generating apparatus of claim 2, wherein the positive electrode and negative electrode of the electrolytic cell are electrically connected to the positive electrode unit and negative electrode unit of the unit cell, respectively.

4. The non-diaphragm neutral sterilizing water generating apparatus of any one of claims 1 to 3, wherein the electrolyzed water tank includes:
an inlet into which the salt water or washing water is introduced; and
an outlet through which the electrolyzed water containing a hypochlorous acid (HOCl) solution generated by electrolyzing the salt water or washing water is discharged.

5. The non-diaphragm neutral sterilizing water generating apparatus of claim 4, wherein the inlet is formed above the outlet.

6. The non-diaphragm neutral sterilizing water generating apparatus of claim 4 or 5, wherein the at least one inlet is formed on a portion opposite to each of two sides of the electrolyzed water tank.

7. The non-diaphragm neutral sterilizing water generating apparatus of any one of claims 4 to 6, wherein the outlet has a filter device.

8. The non-diaphragm neutral sterilizing water generating apparatus of claim 7, wherein the hypochlorous acid (HOCl) solution filtered from the filter device is introduced into the washing water tank or re-introduced into the electrolyzed water tank.

9. The non-diaphragm neutral sterilizing water generating apparatus of any one of claims 1 to 8, wherein the electrolyzed water tank has a bus bar to which the positive electrode and negative electrode of the electrolytic cell are respectively connected, the bus bar being electrically connected to each of the positive electrode unit and negative electrode unit of the unit cell.

10. The non-diaphragm neutral sterilizing water generating apparatus of claim 9, wherein the electrolytic cells of the unit cell are respectively connected to and removed from the bus bar independently from each other.

11. The non-diaphragm neutral sterilizing water generating apparatus of claim 10, wherein the positive electrode and negative electrode of the electrolytic cell are vertically bent, respectively, and the bus bar has a detachable groove for the bent portion to be detached therefrom.

12. The non-diaphragm neutral sterilizing water generating apparatus of claim 11, wherein the electrolytic cell is inserted into and removed from the bus bar in a sliding manner in one direction.

13. The non-diaphragm neutral sterilizing water generating apparatus of any one of claims 1 to 12, wherein a vertically-extending fixing plate is interposed between the electrolytic cells.

14. The non-diaphragm neutral sterilizing water generating apparatus of claim 13, wherein the fixing plate has a plurality of flow holes formed at a predetermined interval to allow the hypochlorous acid (HOCl) solution to flow therethrough.

15. The non-diaphragm neutral sterilizing water generating apparatus of any one of claims 1 to 14, wherein the hypochlorous acid (HOCl) solution generated by the electrolysis performed by the unit cell has a concentration of 300 to 1500 ppm.
